(19) Europäisches Patentamt
European Patent Office
Office européen des brevets

(11) Veröffentlichungsnummer: **0 492 473 A1**

# (12) EUROPÄISCHE PATENTANMELDUNG

(21) Anmeldenummer: **91121863.4**

(51) Int. Cl.5: **C07C 209/68**, C07C 211/45

(22) Anmeldetag: **19.12.91**

(30) Priorität: **20.12.90 CH 4064/90**
**07.05.91 CH 1371/91**

(43) Veröffentlichungstag der Anmeldung:
**01.07.92 Patentblatt 92/27**

(84) Benannte Vertragsstaaten:
**AT BE CH DE DK ES FR GB IT LI LU NL SE**

(71) Anmelder: **LONZA AG**
**Gampel/Wallis Geschäftsleitung Basel**
**CH-4002 Basel(CH)**

(72) Erfinder: **Hardt, Peter, Dr.**
**Bäretstrasse 8**
**Visp (Kanton Wallis)(CH)**
Erfinder: **Völker, Theodor, Dr.**
**Kleestrasse 7**
**Reinach (Kanton Baselland)(CH)**

(74) Vertreter: **Weinhold, Peter, Dr. et al**
**Patentanwälte Dipl.-Ing. G. Dannenberg Dr.**
**P. Weinhold Dr. D. Gudel Dipl.-Ing. S.**
**Schubert Dr. P. Barz Siegfriedstrasse 8**
**W-8000 München 40(DE)**

## (54) Verfahren zur Herstellung von 4-substituierten 2,6-Dialkylanilinen.

(57) Es wird ein neues Verfahren zur Herstellung von 4-substituierten 2,6-Dialkylanilinen der allgemeinen Formel

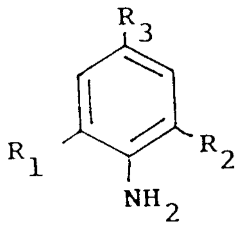

I

beschrieben. Dazu wird ein 2,6-Dialkylanilin mit $\alpha$-Methylstyrol, mit Diisobutylen oder mit Isobuten in Gegenwart eines Katalysators umgesetzt.

EP 0 492 473 A1

Die Erfindung betrifft ein neues Verfahren zur Herstellung von 4-substituierten 2,6-Dialkylanilinen der allgemeinen Formel

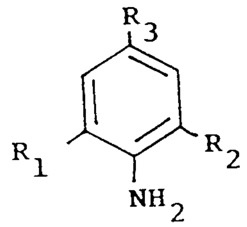

I

worin $R_1$ und $R_2$ gleich oder verschieden sind und ($C_1$-$C_4$)-Niederalkylgruppen, geradkettig oder verzweigt, bedeuten und $R_3$ eine Phenylisopropylgruppe, eine 1,1,3,3-Tetramethylbutylgruppe oder eine tert. Butylgruppe bedeutet.

Die Erfindung umfasst im weiteren die bisher nicht beschriebenen 2,6-Dialkyl-4-(1,1,3,3-tetramethylbutyl)-aniline der allgemeinen Formel

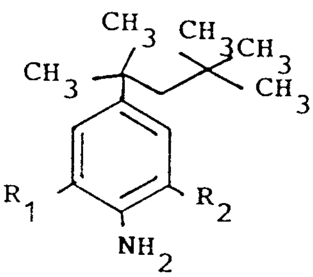

III

worin $R_1$ und $R_2$ die genannte Bedeutung haben, insbesondere das 2,6-Diisopropyl-4-(1,1,3,3-tetramethylbutyl)-anilin mit $R_1$ und $R_2$ = Isopropyl.

Die genannten 4-substituierten 2,6-Dialkylaniline bilden u.a. wertvolle Zwischenprodukte zur Herstellung von Schädlingsbekämpfungsmitteln (EP-A 0 304 025), von Arylthioharnstoffderivaten (DE-OS 27 27 416) oder Hydrolyseschutzmitteln in Polyurethanen (Becher/Braun, Kunststoff Handbuch "Polyurethane", Bd.7, C.Hauser-Verlag 1983, S.407-408).

Aus der EP-A 0 069 065 ist bekannt, aromatische Aniline in para-Stellung mit z.B. $\alpha$-Methylstyrol bzw. mit Diisobutylen zu alkylieren. Diesem bekannten Verfahren ist eigen, dass in Gegenwart einer wässrigen Säure, in der Regel Salzsäure, als Katalysator und unter Zusatz von Zinkchlorid als Co-Katalysator gearbeitet wird. Nachteilig bei diesem Verfahren ist, dass eine beträchtliche Katalysatormenge (Katalysator + Co-Katalysator) von bis zu 2 Molen pro Mol aromatisches Anilin benötigt wird; ausserdem verläuft die Reaktion sehr langsam.

Aus der US-PS 4 340 758 ist z.B. bekannt, 4-t-Butyl-2,6-dimethylanilin über die drei folgenden Stufen in einer Gesamtausbeute von 40% herzustellen. Durch HF-katalysierte Alkylierung von m-Xylol mit Isobuten, in der ersten Stufe, wurde das 5-t-Butyl-1,3-dimethylbenzol erhalten.
Durch weitere Nitrierung mit $HNO_3$ in Gegenwart von Quecksilberacetat wurde das 2-Nitro-1,3-dimethyl-5-t-butylbenzol synthetisiert und schliesslich durch katalytische Hydrierung die Nitrogruppe reduziert.
Die genannte Synthese ist aber einerseits bezüglich der aus ökologischer Sicht und aus prozesstechnischer Sicht problematischen Reaktionsteilnehmer HF und Quecksilberacetat und andererseits bezüglich der schlechten Ausbeuten heutzutags für einen Prozess im industriellen Massstab undenkbar.

In der DE-OS 27 27 416, S.60, wurde vorgeschlagen, das 4-t-Butyl-diethylanilin analog zu Beispiel 9, S.59, durch Ethylierung von 4-t-Butylanilin mit Ethylen in Gegenwart von Aluminiumchlorid bei 200 atm Überdruck und 250 °C zu bilden.
Eine Ausbeuteangabe liegt nicht vor.
Nachteilig bei diesem Verfahren ist, dass neben den drastischen, prozesstechnisch aufwendigen Bedingungen das 4-t-Butylanilin schwer zugänglich ist, d.h. über die Nitrierung von t-Butylbenzol und durch anschliessende Reduktion der Nitrogruppe zum Anilin, hergestellt werden muss. Bekanntlich entstehen bei der Nitrierung von Monoalkylbenzolen zudem Isomerengemische (J.Am.Chem.Soc. 73, 5605 (1951)), die die Zugänglichkeit des 4-t-Butylanilins zusätzlich erschweren.

Umsetzungen des unsubstituierten Anilins mit Isobutylen sind ebenfalls seit langem bekannt. So geht aus der GB-PS 823 223 Example 17, 20 und 49 hervor, dass sich bei der Umsetzung von Anilin mit Isobuten in Gegenwart von Aluminiumchlorid und metallischem Aluminium oder in Gegenwart von Bortrifluorid und unter Druck und bei Temperaturen zwischen 200 °C und 300 °C bei z.T. geringem Umsatz Gemische von Mono- und Diisobutylanilinen ergeben.

Es gelang zwar, mit Festbettkatalysatoren die Umsätze zu verbessern, das Produkt bestand aber weiterhin aus einer Mischung von Mono- und Diisobutylanilinen (EP-A 226 781, EP-A 245 797, Appl. Catalysis 62 (1990) S.161-169).

Die Aufgabe bestand folglich darin, ein Verfahren zu entwickeln, das die Nachteile der bekannten Verfahren nicht aufweist.

Diese Aufgabe konnte auf überraschende Weise mit dem erfindungsgemässen Verfahren gemäss Anspruch 1 gelöst werden.

Ausgangsprodukte bilden die grosstechnisch verfügbaren 2,6-Dialkylaniline der Formel

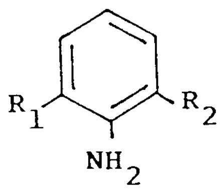

II

worin $R_1$ und $R_2$ gleich oder verschieden sind und ($C_1$-$C_4$)-Niederalkylgruppen, geradkettig oder verzweigt,bedeuten.

Die 2,6-Dialkylaniline werden erfindungsgemäss mit α-Methylstyrol zur Einführung der Phenylisopropylgruppe, mit Diisobutylen zur Einführung der 1,1,3,3-Tetramethylbutylgruppe oder mit Isobuten zur Einführung der tert. Butylgruppe in Gegenwart eines Katalysators umgesetzt.

Geeignete Katalysatoren sind die sogenannten Friedel-Crafts-Alkylierungskatalysatoren, wie sie z.B. in Ullmann's Encyclopedia of Industrial Chemistry 5th ed., Vol. A1, p.185ff beschrieben sind.

Besonders geeignete Katalysatoren sind Aluminiumchlorid oder Bortrifluorid-Etherkomplexe, wie z.B. der Bortrifluorid-Diethyletherkomplex,oder Aluminiumsilikate, wie z.B. Montmorillonit.

Zweckmässig wird der Katalysator in einer Menge von 0,01 bis 0,3 Mol, vorzugsweise von 0,05 bis 0,15 Mol, bezogen auf 1 Mol 2,6-Dialkylanilin, eingesetzt.

Es hat sich herausgestellt, dass eine vorausgehende Aktivierung des Katalysators oder der Zusatz eines Co-Katalysators nicht erforderlich ist.

Die Umsetzung erfolgt zweckmässig bei einer Temperatur zwischen 100 und 300 °C, vorzugsweise zwischen 150 und 250 °C, und bei einem Druck zwischen Normaldruck und 60 bar, vorzugsweise zwischen Normaldruck und 40 bar.

Die Reaktion erfolgt von Vorteil ohne die Gegenwart eines zusätzlichen Lösungsmittels. Zweckmässig fungiert das zum Alkylieren verwendete Olefin, gegebenenfalls im Überschuss angewendet, als Lösungsmittel. Es ist aber durchaus möglich, die Umsetzung in Gegenwart eines inerten Lösungsmittel durchzuführen.

Nach einer Reaktionszeit von üblicherweise 2 bis 20 h können die erhaltenen 4-substituierten 2,6-Dialkylaniline durch einfache Destillation oder durch Salzbildung aus dem Reaktionsgemisch in guter Reinheit und Ausbeute isoliert werden.

Beispiel 1

Herstellung von 2,6-Diisopropyl-4-(phenylisopropyl)anilin

354,6 g 2,6-Diisopropylanilin (2,0 Mol), 239,9 g α-Methylstyrol (2,0 Mol) und 18,62 g Aluminiumchlorid (0,14 Mol) wurden in einem Autoklaven während 4 h auf 205 °C erwärmt (Druck 1 bis 2 bar). Nach dem Abkühlen wurde der Autoklaveninhalt in 600 ml Toluol aufgenommen. Die Toluollösung wurde nacheinander mit 10%iger Natronlauge und Wasser gewaschen und bei Wasserstrahlvakuum und 60 °C Badtemperatur zur Trockene eingedampft.

Es wurden 599,7 g Rückstand mit 73,4% 2,6-Diisopropyl-4-(phenylisopropyl)anilin und 13,4% 2,6-Diisopropylanilin erhalten. Das entsprach einer Ausbeute von 74%, bezogen auf eingesetztes 2,6-Diisopropylanilin. Das Produkt liess sich durch Destillation reinigen (Sdp ca. 150 °C/0,8 mbar; Gehalt 98-99%).

Beispiel 2

Herstellung von 2,6-Diisopropyl-4-(1,1,3,3-tetramethylbutyl)-anilin

59,6 g Diisopropylanilin (0,33 Mol), 3,89 g Aluminiumchlorid (0,03 Mol) und 224,1 g Diisobutylen (78% 2,4,4-Trimethyl-1-penten, 20% 2,4,4-Trimethyl-2-penten) wurden in einem Autoklaven während 15 h auf 200 °C erwärmt (Druck 6 bis 7 bar). Nach dem Abkühlen wurde der Autoklaveninhalt zweimal mit jeweils 100 ml Wasser gewaschen und durch Vakuumdestillation im Rotavapor vom überschüssigen Diisobutylen befreit.

Man erhielt 87,4 g Destillationsrückstand mit 57,5% 2,6-Diisopropyl-4-(1,1,3,3-tetramethylbutyl)anilin und 13,2% 2,6-Diisopropylanilin. Das entsprach einer Ausbeute von 52%, bezogen auf eingesetztes 2,6-Diisopropylanilin.

| | |
|---|---|
| $^1$H-NMR ($CDCl_3$, 300 MHz) $\delta$ in ppm: | 0,68 s, 9H |
| | 1,26 d, J = 6,8Hz, 12H |
| | 1,35 s, 6H |
| | 1,66 s, 2H |
| | 2,94 m, J = 6,8Hz, 2H |
| | 3,57 s, 2H |
| | 7,02 s, 2H |

Beispiel 3

4-t-Butyl-2,6-diisopropylanilin

In einen heizbaren 1l-Rührautoklaven wurden 211,9 g 2,6-Diisopropylanilin (1,14 Mol, Gehalt 95,2%) und 12.1 g Aluminiumchlorid (0,09 Mol) gegeben. Der Autoklav war durch entsprechende Zuleitungen mit einer Stickstoff-Druckflasche und über eine druckfeste Flüssigkeits-Dosierpumpe mit einer Isobuten-Flasche verbunden. Durch mehrmaliges vorsichtiges Aufpressen und Entspannen von ca. 2 bar Stickstoff wurde der Autoklav inertisiert.

Auf gleiche Weise wurde mit Isobuten Stickstoff verdrängt; zuletzt blieb die Reaktionsmischung bei 10 bis 20 °C mit 2 bar Isobuten gesättigt.

Während 50 bis 60 min wurde der Autoklav auf 180 °C aufgeheizt, dabei stieg der Druck auf 28 bis 30 bar, ehe beginnender Druckabfall den Start der Reaktion anzeigte. Beim Druckabfall auf 25 bar wurde jeweils auf 30 bar nachgepresst, bis nach ca. 10 h Sättigung erreicht war.

Der Autoklav wurde auf 20 °C abgekühlt, entspannt und in 250 ml Toluol entleert. Die Toluollösung wurde nacheinander mit 10%iger Natronlauge und Wasser gewaschen und am Rotavapor zur Trockene eingedampft. Man erhielt 293 g Eindampfrückstand enthaltend 90,2% 4-t-Butyl-2,6-diisopropylanilin. Weitere Reinigung erfolgte durch fraktionierende Destillation und Umkristallisation aus Hexan.

| | |
|---|---|
| $^1$H-NMR: ($CDCl_3$, 300 MHz) $\delta$ in ppm | 1,28, d, 12H, J = 6,8Hz; |
| | 1,30, s, 9H; |
| | 2,94, m, 2H, J = 6,8Hz; |
| | 3,62, s, 2H; |
| | 7,07, s, 2H; |
| Siedepunkt: | 154 bis 156 °C/22 mbar |
| Schmelzpunkt: | 73 bis 74 °C |

Beispiel 4

4-t-Butyl-2-ethyl-6-methylanilin

Analog Beispiel 3 wurden 161,7 g 2-Ethyl-6-methylanilin (1,2 Mol, Gehalt 100%) in Gegenwart von 12.1 g Aluminiumchlorid (0,09 Mol) mit 20 bar Isobuten während 20 h auf 200 °C erhitzt und aufgearbeitet. Bei der Vakuumdestillation des Eindampfrückstandes der Toluollösung wurden 182,5 g 4-t-Butyl-2-ethyl-6-methylanilin als farblose, ölige Flüssigkeit erhalten (Gehalt 97,4%; Siedepunkt: 134 °C/15 mbar). Das entspricht einer Ausbeute von 78%, bezogen auf eingesetztes 2-Ethyl-6-methylanilin.

| | |
|---|---|
| $^1$H-NMR: ($CDCl_3$, 300 MHz) $\delta$ in ppm | 1,24, t, 3H, J = 7,5 Hz; |
| | 1,28, s; 9H |
| | 2,17, s, 3H; |

2,52, m, 2H;
3,46, s, 2H;
6,97, s, 2H.

Beispiel 5

4-t-Butyl-2-isopropyl-6-methylanilin

Wie in Beispiel 3 wurden 178 g 2-Methyl-6-isopropylanilin (1,2 Mol, Gehalt 97,3%) in Gegenwart von 12,1 g Aluminiumchlorid (0,09 Mol) mit 30 bar Isobutylen während 4 h bei 220° C gerührt.
Nach Aufarbeitung und Destillation wurden 183,5 g 4-t-Butyl-2-isopropyl-6-methylanilin (Gehalt 97,6%, Siedepunkt 138° C/16 mbar) als farblose ölige Flüssigkeit erhalten. Das entspricht einer Ausbeute von 75%, bezogen auf eingesetztes 2-Methyl-6-isopropylanilin.

$^1$H-NMR: ($CDCl_3$, 300 MHz) $\delta$ in ppm
1,28, s, 9H;
1,28, d, 6H, J = 6,8 Hz;
2,18, s, 3H;
2,91, sept, 1H, J = 6,8 Hz;
3,46, s, 2H;
6,96, d, 1H, J = 1,8 Hz;
7,07, d, 1H, J = 1,8 Hz.

Beispiel 6

4-t-Butyl-2,6-diisopropylanilin

Ein 100 ml-Autoklav wurde mit 17,73 g 2,6-Diisopropylanilin (0,1 Mol), 199 g Toluol, 0,93 g Montmorillonit KSF (Fluka) und 34,3 g Isobuten versetzt und in einem Oelbad während 16 h auf 200° C erwärmt. Der abgekühlte Autoklav wurde entspannt. Die Reaktionslösung enthielt ausser Montmorillonit, Isobuten und Toluol 19,0% 2,6-Diisopropylanilin und 58,3% 4-t-Butyl-2,6-diisopropylanilin.

Beispiel 7

4-t-Butyl-2-ethyl-6-isopropylanilin

Wie in Beispiel 3 wurden 129,0 g 2-Ethyl-6-isopropylanilin (0,79 Mol) in Gegenwart von 8,0 g Aluminiumchlorid (0,06 Mol) mit 30 bar Isobutylen während 6 h bei 200° C gerührt.
Nach Aufarbeitung und Destillation wurden 139,1 g 4-t-Butyl-2-ethyl-6-isopropylanilin (Gehalt 99,3%, Siedepunkt 143° C/17 mbar) als farblose ölige Flüssigkeit erhalten. Das entspricht einer Ausbeute von 80%, bezogen auf eingesetztes 2-Ethyl-6-isopropylanilin.

$^1$H-NMR: ($CDCl_3$, 300 MHz) $\delta$ in ppm
1,27, t, 3H, J = 7,5 Hz;
1,29, d, 6H, J = 6,8 Hz;
1,30, s, 9H;
2,54, q, 2H, J = 7,5 Hz;
2,93, sept, 1H, J = 6,8 Hz;
3,57, s, 2H;
6,99, d, 1H, J = 2,1 Hz;
7,08, d, 1H, J = 2,1 Hz.

Beispiel 8

4-t-Butyl-2,6-di-sec-butylanilin

Wie in Beispiel 3 wurden 20,6 g 2,6-Di-sec-butylanilin (0,1 Mol), 0,59 g Aluminiumchlorid (0,004 Mol) und ca. 38 g Isobuten während 6 h auf 203° C erwärmt.
Nach Aufarbeitung und Destillation wurden 19,5 g 4-t-Butyl-2,6-di-sec-butylanilin als farbloses Oel erhalten (Gehalt 99,6%, Siedepunkt 156° C/17 mbar).
Ausbeute 74%.

$^1$H-NMR: ($CDCl_3$, 300 MHz) $\delta$ in ppm
0,94, m, 6H;

1,28, d, 6H, J = 6,8 Hz;
1,29, s, 9H;
1,56, m, 2H;
1,72, m, 2H;
2,66, m, 2H;
3,56, s, 2H;
6,99, s, 2H.

Beispiel 9

4-t-Butyl-2,6-dimethylanilin

Wie in Beispiel 3 wurden 242,0 g 2,6-Dimethylanilin (2 Mol) in Gegenwart von 24,0 g Aluminiumchlorid (0,18 Mol) während 8 h bei 200 ° C mit 60 bar Isobutylen gerührt.
Nach Aufarbeitung und Destillation wurden 335,7 g 4-t-Butyl-2,6-dimethylanilin (Gehalt 99,8%, Siedepunkt 129 ° C/19 mbar) als farblose ölige Flüssigkeit erhalten. Das entspricht einer Ausbeute von 95%, bezogen auf eingesetztes 2,6-Dimethylanilin.

$^1$H-NMR: ($CDCl_3$, 300 MHz) $\delta$ in ppm
1,27, s, 9H;
2,18, s, 6H;
3,45, s, 2H;
6,96, s, 2H.

Beispiel 10

4-t-Butyl-2,6-diethylanilin

Wie in Beispiel 3 wurden 179,9 g 2,6-Diethylanilin (1,2 Mol) mit 12,0 g Aluminiumchlorid (0,09 Mol) während 12 h bei 200 ° C mit 30 bar Isobutylen gerührt.
Nach Aufarbeitung und Destillation wurden 227,2 g 4-t-Butyl-2,6-diethylanilin(Gehalt 99,7%, Siedepunkt 141 ° C/16 mbar) als farblose ölige Flüssigkeit erhalten. Das entspricht einer Ausbeute von 92%, bezogen auf eingesetztes 2,6-Diethylanilin.

$^1$H-NMR: ($CDCl_3$, 300 MHz) $\delta$ in ppm
1,26, t, 6H, J = 7,5 Hz;
1,29, s, 9H;
2,53, q, 4H, J = 7,5 Hz;
3,51, s, 2H;
6,99, s, 2H.

Beispiel 11

4-t-Butyl-2,6-diisopropylanilin

Wie in Beispiel 3 wurden 17,7 g 2,6-Diisopropylanilin (0,95 Mol, Gehalt 95,2%), 2,0 g Toluol, 2,05 g Bortrifluorid-diehtyletherat (0,013 Mol) und 16,4 g Isobuten (0,29 Mol) während 15 h auf 200 ° C erwärmt.
Der am Schluss der Aufarbeitung erhaltene Eindampfrückstand der organischen Phase (21,3 g) enthielt 75,6% 4-t-Butyl-2,6-diisopropylanilin.

Beispiel 12

2,6-Diisopropyl-4-(1-phenylisopropyl)anilin

177,3 g 2,6-Diisopropylanilin (1 mol) wurden mit 13,38 g $AlCl_3$ (0,1 mol) versetzt und auf 155 ° C erwärmt. Während ca. 6 h wurden 195,8 g $\alpha$-Methylstyrol (2,5 mol) zugetropft. Man liess auf Raumtemperatur abkühlen und versetzte unter Rühren nacheinander mit 300 ml Hexan und 300 ml Natronlauge (20%).
Das Zweiphasengemisch wurde getrennt, die organische Phase mit 900 ml Hexan verdünnt und mit HCl-Gas gesättigt. Der ausgefallene Niederschlag wurde auf einer Fritte gesammelt, mit Hexan gewaschen, gut abgepresst und mit einer Zweiphasenmischung aus 400 ml Toluol und 450 ml Natronlauge (10%) vermischt. Die obere organische Phase wurde abgetrennt und am Rotavapor zur Trockene eingedampft. Man erhielt 288,1 g Eindampfrückstand mit 98,0% 2,6-Diisopropyl-4-(1-phenylisopropyl)anilin, entsprechend

einer Ausbeute von 96%, bezogen auf eingesetztes 2,6-Diisopropylanilin.

$^1$H-NMR: ($CDCl_3$, 300 MHz) $\delta$ in ppm: 6,90, s, 2H; 7,1-7,3, m, 5H; 3,70, s, 2H; 1,12, d, 12H, J = 6,9Hz; 2,91, sept, 2H, J = 6,9Hz; 1,66, s, 6H.

**Patentansprüche**

1. Verfahren zur Herstellung von 4-substituierten 2,6-Dialkylanilinen der allgemeinen Formel

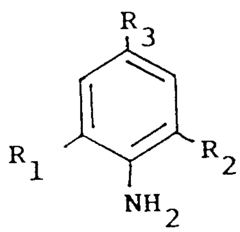

I

worin $R_1$ und $R_2$ gleich oder verschieden sind und ($C_1$-$C_4$)-Niederalkylgruppen,geradkettig oder verzweigt,bedeuten und $R_3$ eine Phenylisopropylgruppe, eine 1,1,3,3-Tetramethylbutylgruppe oder eine tert. Butylgruppe bedeutet, dadurch gekennzeichnet, dass ein 2,6-Dialkylanilin der allgemeinen Formel

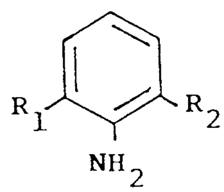

II

worin $R_1$ und $R_2$ die genannte Bedeutung haben, zur Einführung der Phenylisopropylgruppe mit $\alpha$-Methylstyrol, zur Einführung der 1,1,3,3-Tetramethylbutylgruppe mit Diisobutylen und zur Einführung der tert. Butylgruppe mit Isobuten jeweils in Gegenwart eines Katalysators alkyliert werden.

2. Verfahren nach Patentanspruch 1, dadurch gekennzeichnet, dass als Katalysator ein Friedel-Crafts-Alkylierungskatalysator eingesetzt wird.

3. Verfahren nach Patentanspruch 1 oder 2, dadurch gekennzeichnet, dass als Katalysator Aluminiumchlorid, ein Bortrifluorid-Etherkomplex oder Montmorillonit eingesetzt wird.

4. Verfahren nach mindestens einem der Patentansprüche 1 bis 3, dadurch gekennzeichnet, dass der Katalysator in einer Menge von 0,01 bis 0,3 Mol, bezogen auf 1 Mol 2,6-Dialkylanilin, eingesetzt wird.

5. Verfahren nach mindestens einem der Patentansprüche 1 bis 4, dadurch gekennzeichnet, dass die Umsetzung bei einer Temperatur zwischen 100 °C und 300 °C und einem Druck zwischen Normaldruck und 60 bar durchgeführt wird.

6. 2,6-Dialkyl-4-(1,1,3,3-tetramethylbutyl)-aniline der allgemeinen Formel

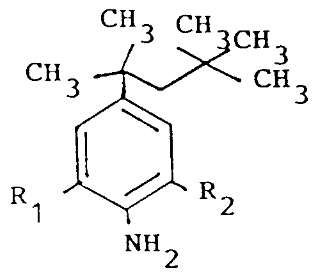

III

worin $R_1$ und $R_2$ die genannte Bedeutung haben.

**7.** 2,6-Diisopropyl-4-(1,1,3,3-tetramethylbutyl)-anilin.

Europäisches Patentamt

# EUROPÄISCHER RECHERCHENBERICHT

Nummer der Anmeldung

EP 91 12 1863

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-0 069 065 (CIBA-GEIGY AG.)<br>* Seite 1 - Seite 4 *<br>* Beispiele 10,12 *<br>* Ansprüche *<br>--- | 1-7 | C07C209/68<br>C07C211/45 |
| A | GB-A-846 226 (FARBENFABRIKEN BAYER AG.)<br>* das ganze Dokument *<br>--- | 1-5 | |
| A | US-A-3 275 690 (R. STROH)<br>* Spalte 1, Zeile 30 - Zeile 34 *<br>* Spalte 2, Zeile 24 - Zeile 29 *<br>* Beispiele *<br>--- | 1-5 | |
| A | US-A-3 714 258 (CH.E BAYHA)<br>* Spalte 3, Zeile 4 - Zeile 46 *<br>----- | 1-5 | |
| | | | **RECHERCHIERTE SACHGEBIETE (Int. Cl.5 )** |
| | | | C07C |

Der vorliegende Recherchenbericht wurde für alle Patentansprüche erstellt

| Recherchenort | Abschlußdatum der Recherche | Prüfer |
|---|---|---|
| DEN HAAG | 23 MAERZ 1992 | PAUWELS G.R.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)